# EUROPEAN PATENT SPECIFICATION

(11) **EP 3 182 878 B1**
(45) Date of publication and mention of the grant of the patent: **17.06.2020**
(21) Application number: 15774694.2
(22) Date of filing: 17.08.2015
(51) Int. Cl.: A61B 3/113, A61B 3/032, A61B 5/16

(54) **APPARATUS FOR DETECTING, DIAGNOSING AND EXERCISING AN INDIVIDUAL'S FUNCTIONALITIES**
VORRICHTUNG FÜR DETEKTION, DIAGNOSE UND TRAINING DER FUNKTIONALITÄTEN EINES INDIVIDUUMS
APPAREIL DE DÉTECTION, DE DIAGNOSTIC ET D'EXERCICE DES FONCTIONS D'UN INDIVIDU

(30) Priority: 21.08.2014 IT TO20140676
(43) Date of publication of application: 28.06.2017
(73) Proprietor: Cagno, Andrea, 10042 Nichelino (Torino) (IT)
(72) Inventor: Cagno, Andrea, 10042 Nichelino (Torino) (IT)
(74) Representative: Baldi, Claudio
(86) International application number: PCT/IB2015/056243
(87) International publication number: WO 2016/027215

(56) References cited:
- WO-A1-2008/139137
- WO-A1-2009/053917
- US-A1- 2005 177 065
- US-A1- 2012 293 773

## Description

### Technical Field

The present invention relates to an apparatus for detecting, diagnosing and exercising an individual's functionalities. More particularly, the invention relates to an apparatus for detecting, diagnosing and exercising the functionalities of visual, visuo-motor, auditory, audio-motor, visuo-verbal, audio-verbal brain areas and for the oculo-audio-motor coordination of an individual.

### Prior Art

Apparatuses for detecting, diagnosing and exercising the functionalities of visual brain areas in individuals are known in the medical fields of ophthalmology and rehabilitation. These apparatuses usually comprise an emitting device adapted for stimulating one of the senses of an individual, typically his/her sight, and a sensing device adapted for generating an electric signal indicative of the individual's reaction induced by activation of the emitter. Examples of known apparatuses of this kind are described in US2011184498 (A1), WO 2007/142588 and US2010/216104.

US 2005/177065A1 on which the preamble of claims 1 and 4 is based discloses an apparatus for detecting, diagnosing and exercising an individual's functionalities.

Other similar apparata for detecting, diagnosing and exercising an individual's functionalities are also disclosed in WO 2009/053917 A1, WO 2008/139137 A1 and US 2012/293773 A1.

The known apparatuses, although they sometimes make use of sophisticated devices, such as for instance monitors of the "touch screen" type, are however rudimentally controlled and they do not give satisfactory results when the functionalities of an individual, particularly those of an athlete, are to be detected, diagnosed and exercised.

The main object of the invention is to provide a solution to the problem of how to improve detecting, diagnosing and exercising the functionalities of visual, visuo-motor, auditory, audio-motor, visuo-verbal, audio-verbal brain areas and to improve the oculo-audio-motor coordination of an individual.

A further object of the invention is to provide an apparatus that can be industrially manufactured and can be used, especially by physicians, eye specialists and technicians working in the fields of ophthalmology, motor sciences, orthoptics, optometry, postural rehabilitation, neurology and speech therapy.

A particular object of the invention is to provide an apparatus and a method for controlling such apparatus that are effective in detecting, diagnosing and exercising an athlete's functionalities.

A not least object of the invention is to provide an apparatus and a method that allow to detect, diagnose and exercise the functionalities of visual brain areas and, simultaneously, motor performances.

### Summary of the Invention

These and other objects of the invention are achieved with the apparatus and method as claimed in the appended claims.

The solution proposed according to the invention provides for stimulating, by means of emitters of optical sensorial stimuli, an individual's ocular reaction. In a preferred embodiment of the invention, the emitters are distributed on a plane in a matrix form. The emitters are activated according to a predetermined sequence so as to define a path visible in space. The path is particularly defined by a first emitter or "start" emitter, by a series of intermediate emitters or "progress" emitters, and by a last emitter or "stop" emitter. In addition, the sequence and consequently the definition of the corresponding path can be repeated many times. According to the invention, the apparatus comprises a stock of activation sequences for the emitters and each sequence preferably comprises at least three emitters, including a "start", a "progress" and a "stop" emitter. The apparatus further performs the predetermined sequence and activates the emitters according to one or more parameters. Said parameters modify the sensorial effect of the performance of the sequence by the apparatus. They are for instance represented by values of time interval according to which the emitters of the sequence are activated, or by values of emission intensity of the emitters, for instance a more or less intense brightness. The same sequence may for instance be performed more or less rapidly, or the brightness emitted by the emitters may be more or less intense depending on the selected parameter. The parameters governing activation of the emitters further determine the direction of movement along the path. In addition, the parameter may be unique for the entire sequence, or different steps of the sequence may be performed with different parameters.

Optical stimuli may be distributed along rectilinear or curvilinear paths, on vertical or horizontal surfaces, in a plane, whereby they may have bi-dimensional distribution, or in space, whereby they may have a tri-dimensional distribution. In addition, visual stimuli may be accompanied by acoustic stimuli generated by sound emitters.

Light emitters can have different shapes, for instance circular, made of symbols, letters, numbers, arrows, and different colors.

The emitters may further be associated to representations in graphics or with corresponding colors, arranged on surfaces that are visible and/or physically accessible by individual users and separated from the area in which the emitters are located. For instance graphic representations of the same symbols as those of the emitters can be represented on treadable surface such as mats, platforms or panels that are arranged vertically and can be reached by the user's extremities.

In a particular embodiment of the apparatus, there can be provided proprioceptive tools capable of stimulating the capacity of perceiving and recognizing the position of one's own body in space and the state of contraction of one's own muscles. Proprioceptive tools, such as for instance a vestibular board (either monopodalic or bipodalic) or a proprioceptive platform, are capable of creating a further difficulty for the user and stimulating balance capacity.

According to the invention, the stimuli are of optical type and the audio stimuli that may be provided function as a support for optical stimuli. Specifically, the audio stimuli can provide sound support in path identification. In one embodiment of the invention, audio stimuli are emitted in advance of the activation of optical emitters, so as to promote the user's response for obtaining correct detection, diagnosis and exercise of the functionalities of a user's brain areas. As an alternative, audio stimuli are synchronized with the activation of optical emitters. As another alternative, audio stimuli are delayed or randomized with respect to the activation of optical emitters, so as to intentionally create a situation of confusion for the individual.

The apparatus according to the invention comprises an ocular tracker "Eyetracker" for detecting monocular or binocular fixation. The tracker has the function of monitoring, quantifying and qualifying the movements of the user's eye, detecting delays and/or advances, errors and/or omissions with respect to optical stimuli. The tracker can also consist of glasses of the "Eyetracker" type.

In addition, there can be provided so-called "wearable devices" such as socks, shoes, T-shirts etc. having miniaturized computers and sensors that are mounted on board and are capable of analyzing the movements of the user's body or other functions such as heartbeat etc.

The apparatus may also be associated to directional references, i.e. visible references arranged as "targets" to which the user must direct his/her body or direct the movement of his/her limbs, for instance in order to simulate hitting against a ball.

At the beginning of the working cycle of the apparatus, all emitters are preferably in a deactivated condition, i.e. they do not generate the sensorial stimulus perceivable by the individual, who therefore is not able to identify in advance the path defined by the sequence. At this stage, the individual is in a stand by state, but he/she can be standing still or in motion, for instance depending on the conditions of the test or exercise to be performed.

Emitters may consist of physical emitters, such as light bulbs or LEDs or luminescent tubes or portions of a display or monitor for instance connected to a computer, or of ephemeral emitters generated by projecting symbols or shapes on a wall or floor. Emitters can have various shapes and also consist of light trails or holograms.

The apparatus optionally comprises one or more motion sensors that can also consist of push-buttons or touch sensors. The sensors have the function of monitoring, quantifying and qualifying the user's movements, detecting delays and/or advances, errors and/or omissions with respect to eye-hand, eye-foot, eye-body coordination. Such sensors can be stationary, for instance infrared sensors, ultrasonic sensors, pressure sensors etc., or they can be incorporated in a suitable equipment and used and/or worn by the user, for instance accelerometers, gyroscopes etc. The apparatus may further comprise one or more voice-controlled sensors.

The apparatus can adopt a specific customized protocol for detecting, diagnosing and exercising an individual's functionalities, particularly visual and motion functionalities. The apparatus monitors the user's response speed and adapts itself correspondingly, by adopting different sequences, for instance more easy or difficult for the user, as well as different parameters such as sequence performing speed, activation direction of the emitters, distance between the emitters, sequence repetition frequency. The apparatus optionally provides a control for manually selecting sequences, for instance by an operator or by the individual using the apparatus.

Advantageously, owing to the fact that the predetermined sequence with which the emitters are activated comprises the references of a group of emitters arranged along a path indicative of the beginning, end and progression of movement in space, and owing to the fact that said emitters are activated according to a sequence reproducing the actual eye movement to be promoted, it is possible to improve the visual and motion performances of an individual. Preferably, according to the invention, the path is not perceivable by the individual in advance of the activation of the emitters associated thereto.

### Brief Description of the Drawings

Some preferred embodiments and alternatives of the invention will be described below by way of non-limiting examples with reference to the annexed drawings, in which:
- Figure 1 shows the apparatus according to a preferred embodiment of the invention in a first operating configuration;
- Figures 2-4 are schematic views of the apparatus of Fig.1 in corresponding operating configurations;
- Figure 5 shows the apparatus according to a first alternative;
- Figure 6 shows the apparatus according to a second alternative;
- Figures 7A to 7F show the apparatus in corresponding embodiments;
- Figures 8 and 9 show the apparatus in further corresponding embodiments.

### Description of a Preferred Embodiment of the Invention

In the annexed figures, same or functionally equivalent parts are designated with the same reference numerals. In the annexed figures, the apparatus according to the invention has been indicated as a whole with reference numeral 11. This is an apparatus for detecting, diagnosing and exercising functionalities of an individual, who is illustrated and identified in the figures with the reference numeral 13.

The apparatus 11 comprises a plurality of emitters 15 emitting optical stimuli perceivable by an individual. The emitters 15 comprise light emitters such as lamps or LEDs and may comprise also sound emitters. The emitters 15 are preferably distributed in a matrix form on a flat board 17 made of a suitable material, for instance wood or plastics. The emitters 15 can be evenly distributed at the intersections between the rows and the columns of the matrix, or they can be distributed according to other arrangements. The emitters 15 can also be located on different planes and define as a whole a tridimensional matrix.

The apparatus 11 comprises a unit 19 controlling the activation of the emitters 15. The unit 19 is programmed for activating the emitters 15 according to a predetermined sequence Q and according to at least one parameter P₁,P₂,...Pₙ. Depending on the spatial arrangement of the emitters 15, the activation sequence for the emitters 15 originates a different path on the plane or in space. The path is adapted to stimulate and guide eye movement of an individual who can perceive the sensorial stimuli emitted by the emitters 15.

The apparatus 11 further comprises a memory unit 21 in which predetermined activation sequences Q₁,Q₂,...Qₙ for said emitters 15 as well as the parameters P are stored. Each sequence is identified by a univocal sequence reference and comprises the references of a group of emitters 15 belonging to the plurality of emitters. The references of the emitters 15 of the group of emitters may consist for instance of univocal codes identifying the emitters, for example 001, 002,...00n, or of spatial coordinates of the emitters, for example x₁,y₁, x₂,y₂,...xₙ,yₙ.

In addition, preferably, the sequences Q stored in the memory unit 21 are classified on the basis of a grade of difficulty in performance for an individual, for instance an athlete practicing a specific sports discipline such as tennis.

The emitters 15 belonging to each sequence are arranged along a path corresponding to the eye movement to be induced in the individual user 13. The activation of the emitters 15 along a path determined by the selected sequence may further also induce displacement in space of the individual's body or part thereof.

The apparatus 11 comprises a monocular or binocular sensor 31 "Eyetracker" adapted to detect eye movements and capable of generating an electrical signal indicative of the eye movements of an individual 13. The sensor 31 is preferably of the binocular type, i.e. capable of detecting the eye movements of both eyes. The sensor 31 can be of the wireless type or wired type. In addition, the sensor 31 may be associated to the board 17 or separated from it. In other embodiments the sensor 31 may consist of glasses of the "Eyetracker" type.

The apparatus 11 comprises a processing unit 25 operationally connected to said control unit 19. According to the invention, the processing unit 25 is programmed for processing the signal coming from the sensor 31 and for selecting, depending on the value taken by said signal, the value of said at least one parameter P and a predetermined sequence out of said sequences Q stored in the memory unit 21.

In a preferred embodiment of the invention, the control unit 19, the memory unit 21 and the processing unit 25 are part of an electronic processor or personal computer.

Still according to the invention, the value taken by the parameter P and/or the predetermined sequence to be performed in a subsequent cycle of performance of a sequence are selected depending on the individual's performances in the preceding cycle. More precisely, the parameter or parameters P and/or the sequence Q of the cycle to be performed is/are selected based on the time Tc, measured in the preceding cycle and elapsed between the activation of an emitter 15 and the moment at which the individual's eyes become fixed on the activated emitter 15, for instance the first "start" emitter or the last "stop" emitter of the sequence, or become fixed on another emitter 15, activated subsequently, of the sequence, for instance one of the "progress" emitters 15. Therefore, according to the invention, there is provided to measure at least one time Tc, and preferably of a plurality of times Tc, during performance of a sequence Q. The moment of activation of the emitter 15 is known, being associated to each sequence Q and depending on the parameter P. The time Tc corresponds to the time elapsed between the activation of the emitter 15 in the sequence Q and the end of the eye movement signaled by the motion sensor 31 corresponding to the moment when the eyes become fixed on the corresponding emitter 15. The time Tc depends on the initial positions of the individual's body and eyes, such positions being dictated for instance by a rule associated to the sequence Q or being random and freely established by the individual.

In the processing unit 25, the signal coming from the eye motion sensor 31 is processed for calculating the time Tc. The time Tc is used by the processing unit 25 for selecting the value of the parameter P and/or the sequence Q to be used in the subsequent working cycle of the apparatus 11.

According to the invention, the processing unit 25 is programmed for selecting the parameter P and/or the sequence Q so as to detect, diagnose and exercise the functionalities of an individual 13 using the apparatus. The processing unit 25 is preferably programmed for selecting the parameter or parameters P and/or the sequence Q so as to promote progressive improvement in the visual and motion performances of the individual user. In the latter case the processing unit 25 is preferably programmed for selecting the value of the parameter P and/or the predetermined sequence Q so as to minimize the time Tc.

In a preferred embodiment of the invention, the processing unit 25 is programmed for operating according to one of the following three modes. In the first mode only the parameter P is varied, whereas the sequence Q remains the same as in the preceding cycle, in the second mode only the sequence Q is varied and the parameter P remains the same as in the preceding cycle, and in the third mode both the parameter P and the sequence Q are varied. In the first mode, when Tc exceeds a predetermined threshold S₁ (T_{C} > S₁) the at least one parameter P determining the performing time for the sequence is varied so as to reduce the sequence performing speed and the same sequence is preferably performed as long as Tc = S₁. This situation corresponds to the case in which the eyes of the individual user have moved more slowly with respect to the time with which the emitters of the sequence are activated and therefore the eyes need to be trained starting from a performing speed closer to their performances. Instead, when the time Tc is lower than the predetermined threshold (Tc < Si), the parameter P determining the sequence performing speed is varied so as to increase the sequence performing speed and the same sequence is preferably performed until Tc = S₁ is reached. This case corresponds to the situation in which the individual's eyes have moved more rapidly with respect the sequence performing time. In the second mode, when Tc exceeds a predetermined threshold S1, the sequence Q selected for the subsequent cycle will have a difficulty grade lower than that of the performed sequence, i.e. a difficulty more appropriate to the user's performances. Instead, when Tc is lower than the predetermined threshold SI, a sequence of higher difficulty than that of the formerly performed sequence is selected . In the third mode, the functionalities of the first and second modes are combined, and preferably the parameter P and the sequence Q are selected so as to induce, in the individual 13 using the apparatus, a progressive improvement of the visual performances and possibly of the motion performances, so that Tc = S₁ is reached.

The apparatus 11 may further be equipped with a body motion sensor 23 capable of generating an electrical signal indicative of the variation of the position of a body movable in space and therefore of providing a signal indicative of the beginning and end of a body movement. The motion sensor 23 can be of various types and capable of providing a continuously variable signal, depending on the position taken in space, or a signal indicative of an occurred displacement of the movable body 13. The motion sensor 23 may comprise for instance an infrared sensor, an ultrasonic sensor, a pressure sensor, a gyroscope or an accelerometer or even a simple push-button or touch sensor. The sensor 23 can by of the wireless type or of the wired type. The sensor 23 may further be associated to the moving body or be separated from it. In the first instance, the sensor 23 can comprise for instance a strap for being fixed to an individual's wrist or ankle. In the second instance, the sensor 23 can be fixed to a vertical wall or a horizontal surface or also be incorporated in a mat or in a cushion. The motion sensor 23 provides a signal indicative of the variation of the position of a body movable in space. The motion sensor 23 can be of various types and capable of providing a continuously variable signal, depending on the position taken in space, or a signal indicative of an occurred displacement of the movable body 13.

The sensor 23 provides a signal indicative of the variation of the position of a body movable in space to the processing unit 25 and therefore allows to calculate the time Ts elapsed between the activation of an emitter 15, for instance the first "start" emitter or the last "stop" emitter of the sequence or also one of the "progress" emitters, and the moment at which the individual's movement begins or a specific movement established by the exercise which is being performed, for instance a step forward, a side jump, a hit with a tennis racket, is detected. The individual's movement is signaled by the motion sensor 23. It is clear that the time Ts depends on the individual's initial position and on whether the individual is in motion or is standing still. The processing unit 25 measures the time Ts and compares it with a threshold S₂. The result of the comparison is used by the processing unit 25 for selecting the parameter P and the sequence Q to be used in the subsequent working cycle of the apparatus 11. The selection preferably takes place in the same ways as those described with reference to times Tc. According to the invention, it is possible to provide for measuring a plurality of times Ts during performance of a sequence Q.

Still according to the invention, it is also possible to provide that the selection of the parameter P and of the sequence Q to be used in the subsequent working cycle of the apparatus 11 takes place depending on the difference between the times Ts and Tc. This difference is indicative of the speed at which the individual makes a movement with his/her body when such movement is induced by the seeing of an optical stimulus.

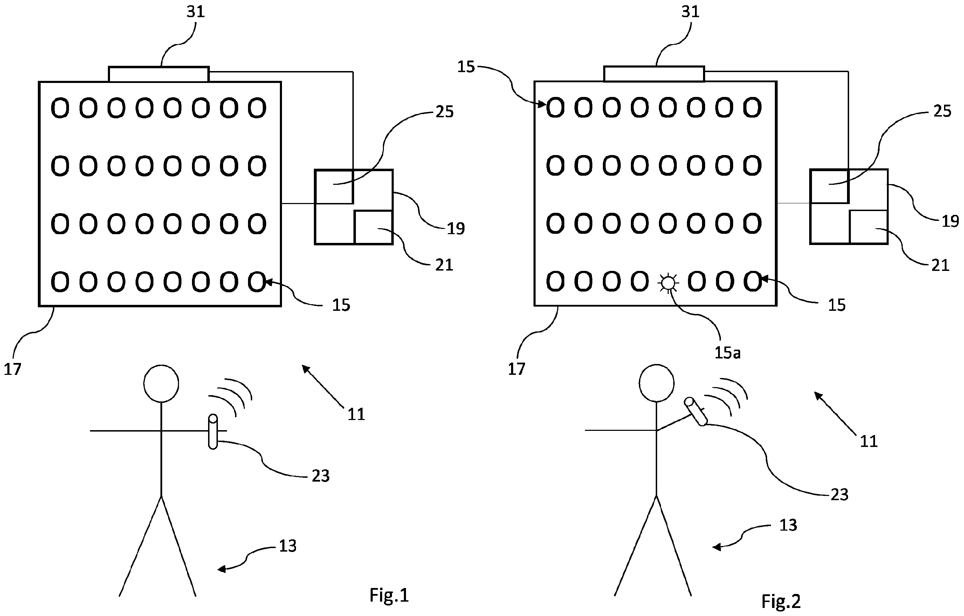

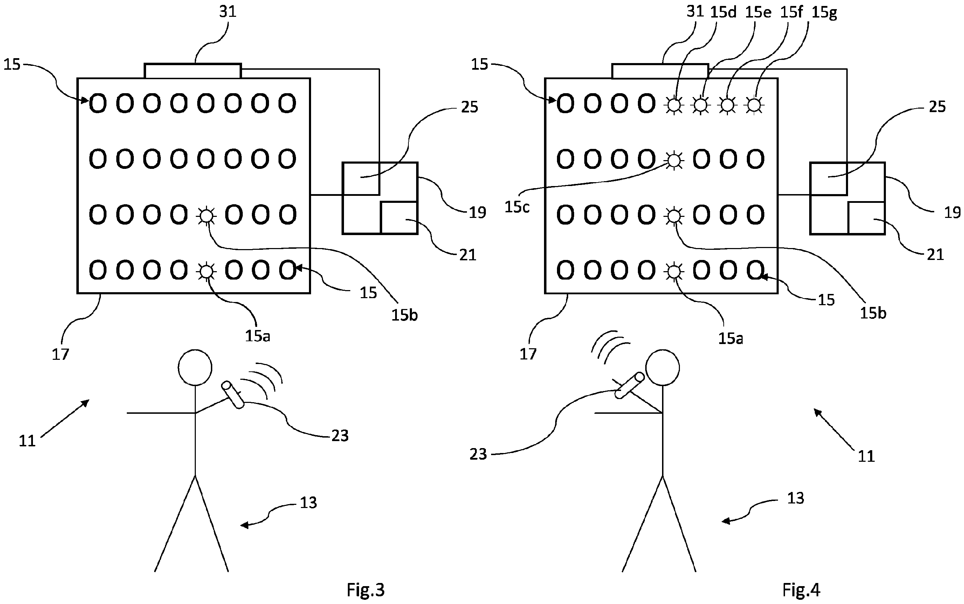

Referring now to Figure 1, there is illustrated an apparatus 11 according to a preferred embodiment of the invention. The apparatus 11 comprises a board 17 on which a plurality of emitters 15 of the light-emitting type, for instance LED emitters, are provided. The emitters 15 are distributed in a matrix form and, in the shown example, comprise four rows and eight columns. Figure 1 refers to the starting configuration of the apparatus 11 in which all the emitters are in a deactivated condition. The individual 13 using the apparatus 11 stands in front of the board 17, in a starting position, and wears a motion sensor 23 associated to his/her right wrist. At the beginning of the working cycle of the apparatus 11, the control unit 19 performs a first predetermined sequence. The first sequence performed by the apparatus 11 when switched on may correspond to a randomly selected sequence, out of the sequences stored in the memory nit 21, or to a specific predetermined starting sequence, or to the last sequence performed by the apparatus before being switched off. Referring to Figure 2, there is illustrated the configuration corresponding to the activation of a first emitter 15a in the sequence performed by the control unit 19. In the shown example, said first emitter 15a is the fifth emitter from the left in the first row from the bottom of the board 17. Referring to Figure 3, there is illustrated the configuration corresponding to the activation of a second emitter 15b in the sequence. The second emitter 15b is the fifth emitter from the left in the second row from the bottom of the board 17.

Referring to Figure 4, there is illustrated the configuration corresponding to the activation of all the emitters 15a, 15b, 15c, 15d, 15e, 15f, 15g of the sequence. In the shown example, the sequence comprises in total seven emitters and represents, on the plane of the board 17, two segments of straight line arranged at 90° to form a path having a shape like an L turned upside down. The emitters 15 are activated progressively and are maintained activated until the end of the sequence performance. In addition, the time interval elapsing between the activation of an emitter 15 and the activation of the subsequent emitter and being determined by a corresponding parameter P corresponds to a sub-multiple of the total performing time of the sequence. In other embodiments it will be possible to provide that the time intervals between the activation of an emitter 15 and the activation of the subsequent emitter are determined by corresponding mutually different parameters P and that consequently the sequence progression speed is not constant.

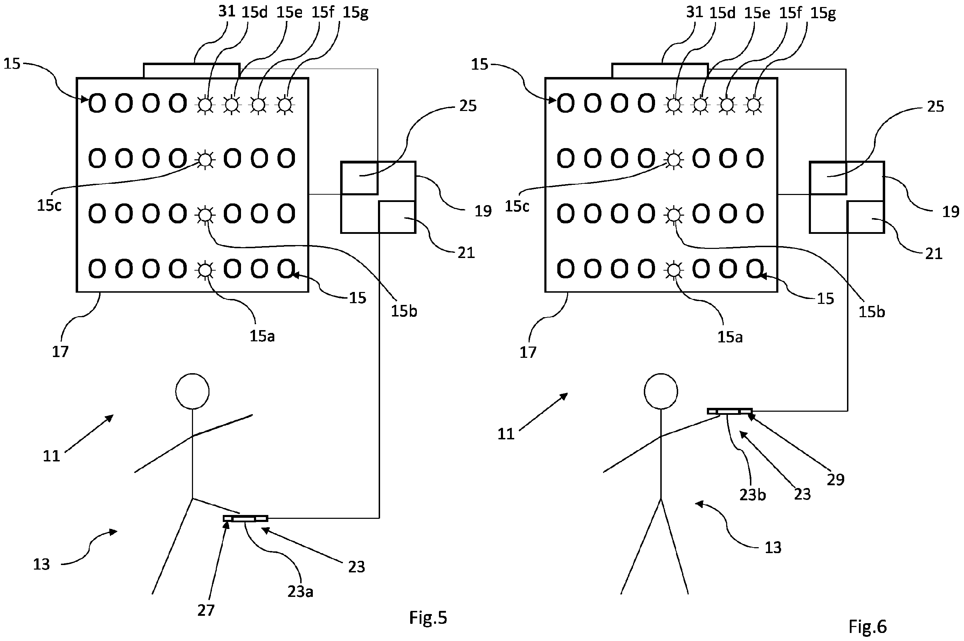

Referring to Figure 5, there is illustrated a first alternative of the apparatus 11 in which the motion sensor 23 comprises a pressure sensor 23a located in a platform 27. When the platform 27 is treaded by the user's foot, the signal transmitted by the sensor 23a to the processing unit 25 is processed and the time Ts elapsed between the activation of an emitter 15, for instance the first emitter 15a in the sequence Q, and the arrival of the signal at the processing unit 25 is measured and used for selecting the sequence Q and the parameter P of the subsequent working cycle of the apparatus 11.

Referring to Figure 6, there is illustrated a second alternative of the apparatus 11 in which the motion sensor 23 comprises a pressure sensor 23b located in a push-button 29. When the push-button 29 is pressed by the hand of the user 13, the signal transmitted by the sensor 23b to the processing unit 25 is processed and the time Ts elapsed between the activation of an emitter, for instance, the first emitter 15a in the sequence and the arrival of the signal at the processing unit 25 is measured and used for selecting the sequence Q and the parameter P of the subsequent working cycle of the apparatus 11.

According to the invention, the emitters 15 can have substantially any shapes and arrangements. In addition, the emitters 15 can be associated to corresponding symbols which are distributed in space and with which the individual 13 must interact when the emitters 15 in the board 17 are activated.

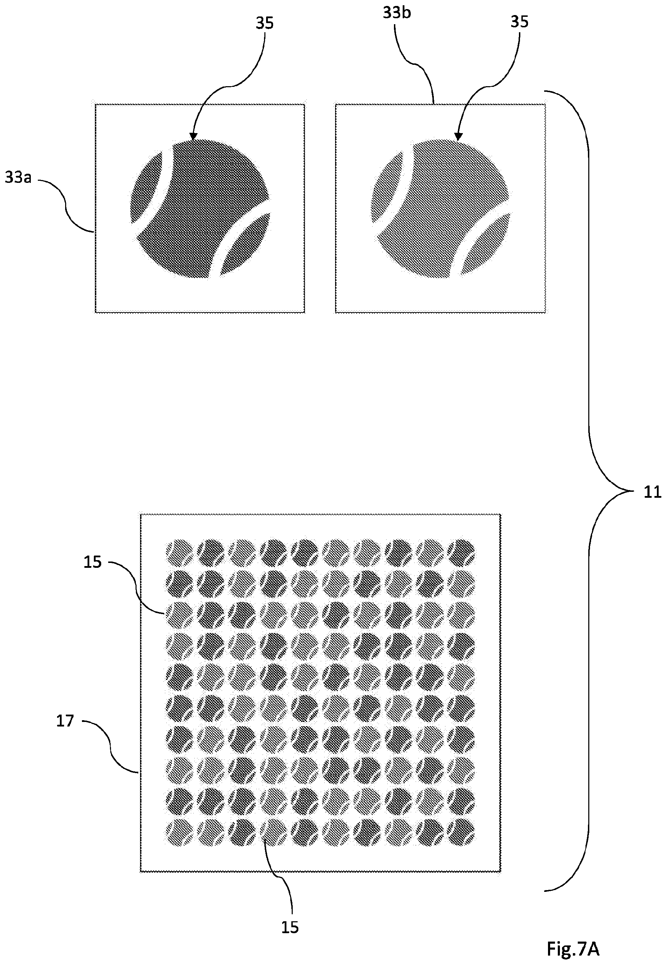

Referring to Figure 7A, there is illustrated an example of board 17 in which the emitters 15 are distribute in a matrix form and represent a tennis ball. A pair of corresponding mats or platforms or vertical panels 33a,33b on which a corresponding tennis ball 35 is represented, are provided for guiding the eye or body movements of the individual's body 13 when a sequence Q of activation of the emitters 15 is performed on the board 17. For example it may be provided that upon each activation of an emitter 15 on the board 17, the individual 13 places himself alternately on one of the represented symbols 35. The symbols 35 are preferably associated to a pressure sensor of the kind described above and indicated with the reference numerals 23a or 23b.

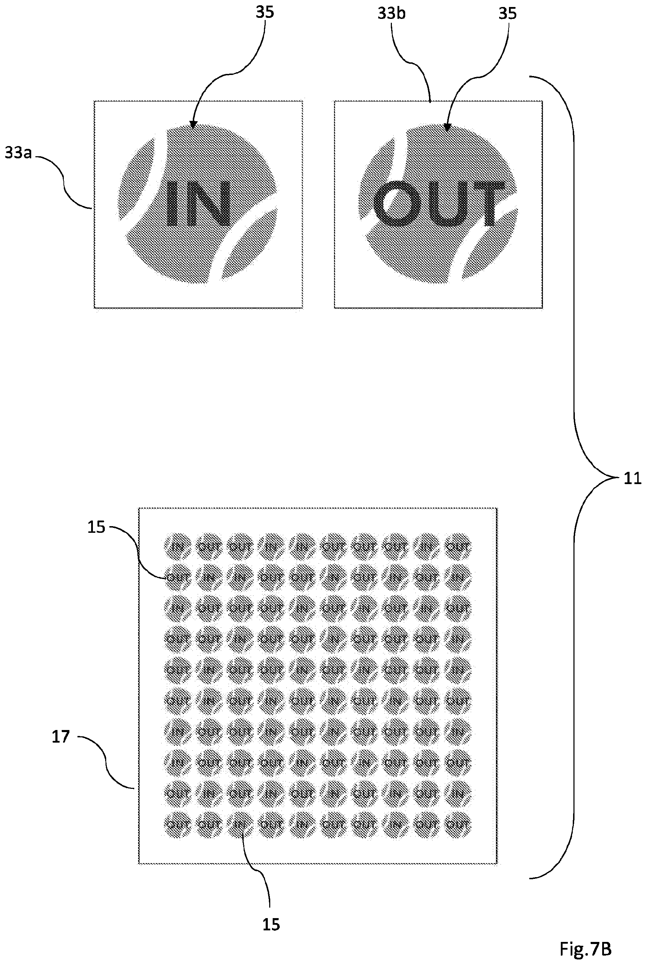

Referring to Figure 7B, the emitters 15 show two graphic symbols "IN" and "OUT" randomly distributed in a matrix form on the board 17. A pair of corresponding mats or platforms or vertical panels 33a,33b showing identical symbols 35 are provided for guiding the eye or body movements of the individual 13 when an activation sequence for the emitters 15 is performed on the board 17. For instance, it is possible to provide that upon each activation of an emitter 15 on the board 17, the individual 13 fixes his/her eyes or places himself/herself with his/her body, for instance with his/her feet, on the corresponding shown symbol 35, and in the latter case the symbols 35 will preferably be associated to a pressure sensor 23a or 23b.

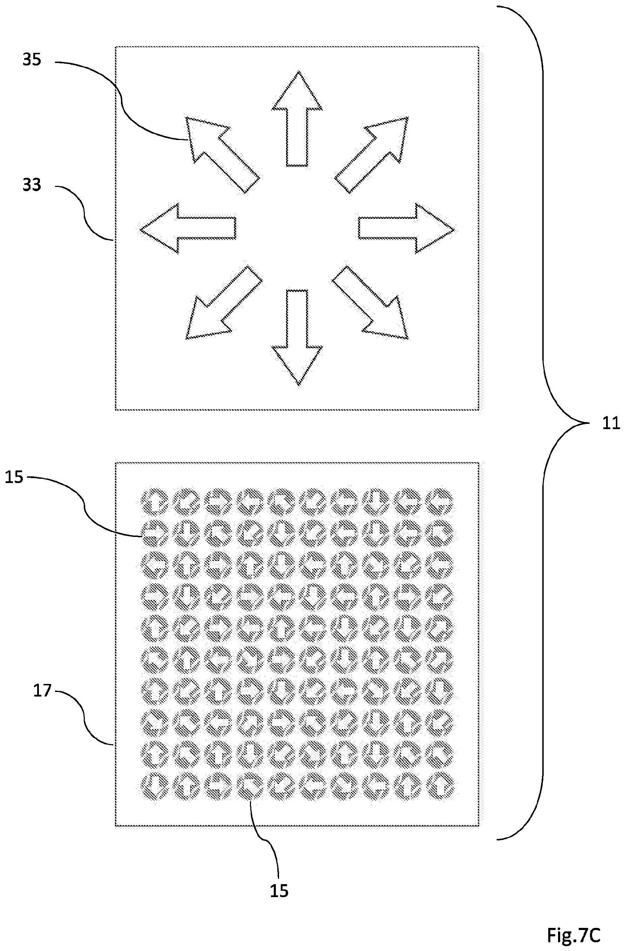

In Figure 7C, the emitters 15 show corresponding arrows and are distributed in a matrix form with the arrows oriented along different directions on the board 17. A corresponding mat or platform or vertical panel 33 showing a set of arrows 35 arranged in a sunburst manner is provided for guiding the eye or body movements of the individual 13 when an activation sequence for the emitters 15 is performed. For instance, it is possible to provide that upon each activation of an emitter 15 on the board 17, the individual moves his/her body and/or directs his/her eyes along the corresponding arrow symbol 35 shown on the mat or platform or vertical panel 33. In this case, too, the symbols 35 will preferably be associated to a pressure sensor of the 23a or 23b type.

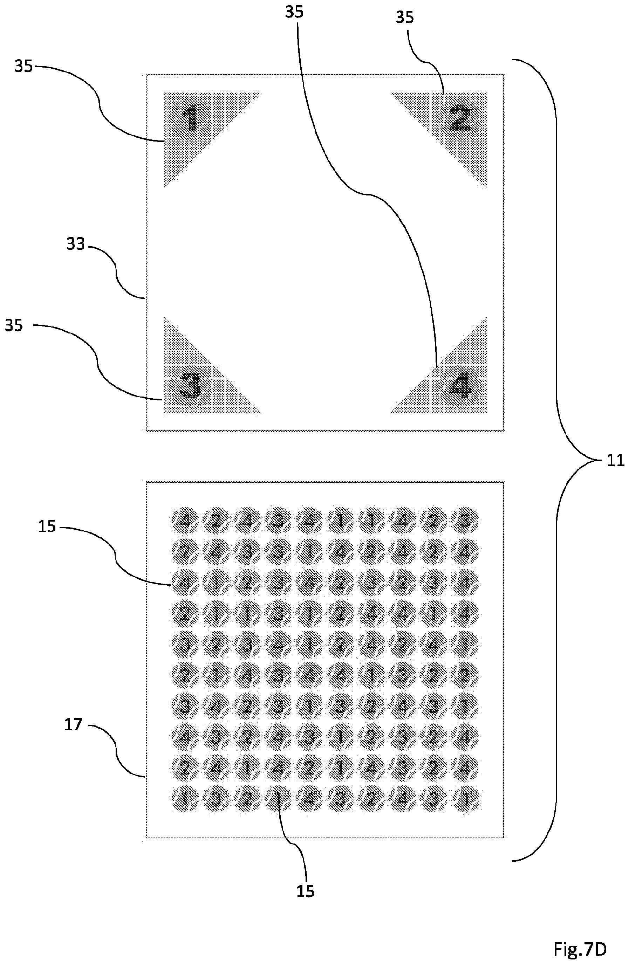

In Figure 7D, the emitters 15 show four graphic symbols corresponding, in the shown example, to the digits "1" to "4" and are randomly distributed in a matrix form on the board 17. A corresponding mat or platform or vertical panel 33 showing corresponding graphic symbols 35 is provided for guiding the eye or body movements of the individual 13 when an activation sequence Q for the emitters 15 is performed on the board 17. For instance it is possible to provide that upon each activation of an emitter 15 on the board 17, the individual places himself/herself with his/her feet on the corresponding symbol 35. In the shown example, the symbols 35 are arranged each at the four corners of the mat or platform or vertical panel 33. The symbols 35 are further preferably associated to a pressure sensor of the 23a or 23b type.

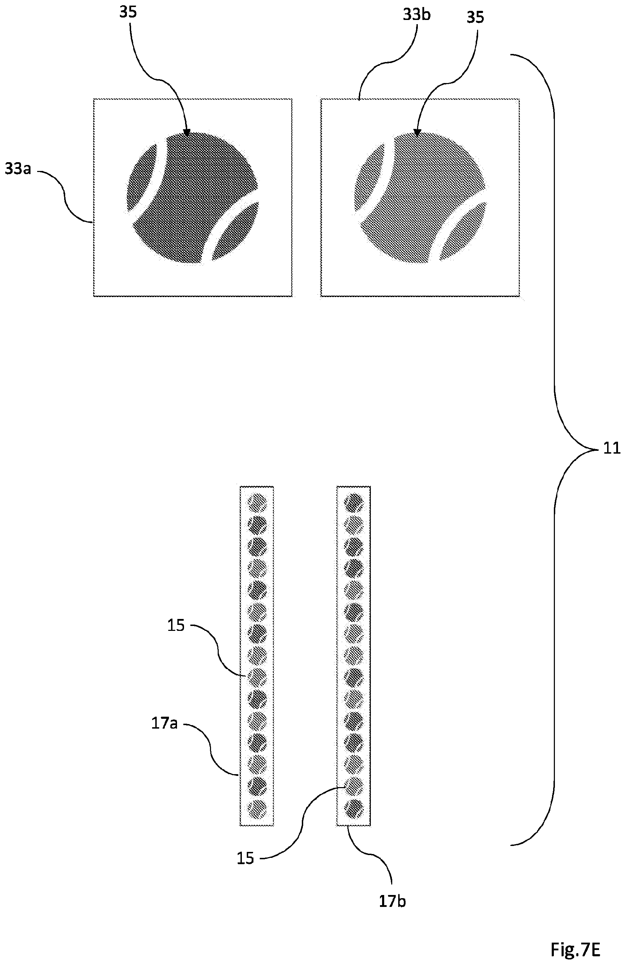

In Figure 7E there is illustrated a configuration which is similar to the one of Figure 7A, but in which the emitters 15 are arranged on a pair of boards 17a,17b. Each board 17a,17b comprises a row of emitters 15 which, when activated, show each a tennis ball.

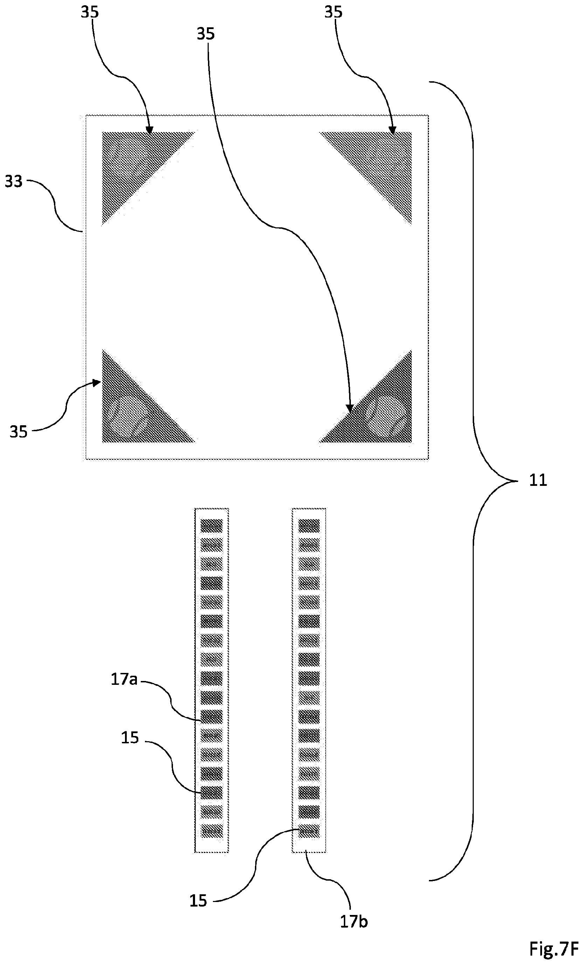

In Figure 7F there is represented a configuration of the emitters 15 which is similar to the one of Figure 7E, but in which the emitters 15, when activated, show symbols identifying four different colors, each symbol being associated to a color name that does not match the color of the symbol. Each board 17a,17b comprises a row of emitters 15. A corresponding mat or platform or vertical panel 33 showing four graphic symbols 35 corresponding to the angles is provided for guiding the eye or body movements of the individual 13 when an activation sequence Q for the emitters 15 is performed on the boards 17a,17b. For instance, it is possible to provide that upon each activation of an emitter 15 on the board 17 the individual positions himself/herself with his/her feet on the symbol 35 corresponding to the color of the activated emitter 15 or to the color name appearing in the activated emitter 15.

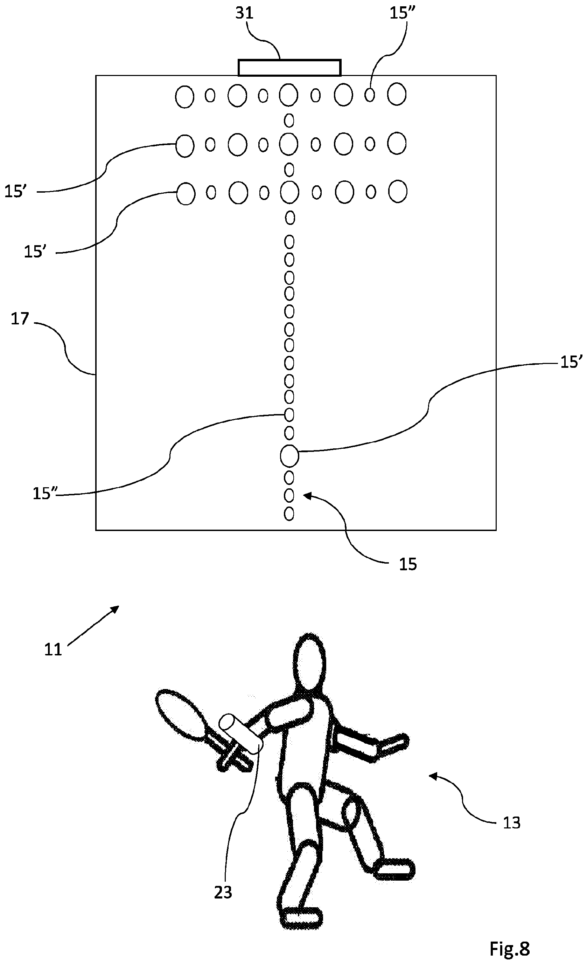

Referring to Figure 8, there is illustrated a second embodiment of the apparatus 11 according to the invention. The apparatus 11 of Figure 8 is intended especially for training athletes practicing tennis. The apparatus of Figure 8 is further intended especially for training service return in playing tennis. In this embodiment the apparatus comprises a plurality of light emitters 15 distributed on a vertical board 17. The emitters 15 are arranged in a cross-like pattern, the stem of the cross being defined by a single row of emitters and the arms of the cross being defined by three rows of emitters. Some of the emitters 15 have a size larger than the other ones. These larger emitters 15 are arranged for signalling to the user particular moments in the sequence. In the illustrated apparatus, the moment in which the ball is released by the rival is signaled by a first larger emitter 15' located in the fourth position from the bottom in the sequence. The moment at which the ball is hit by the serving player is signaled by larger emitters 15' arranged along the arms of the cross. In the shown example, each side of the arms of the cross has six larger emitters 15' distributed in three parallel rows, two emitters 15' for each of the three rows, alternated with emitters 15" of smaller size with respect to the emitters 15'. Each horizontal row of emitters further comprises a central larger emitter 15'. In this embodiment, the sequences Q stored in the memory unit 21 differ in the height the ball reaches when hit, in the direction from which the ball is hit, right or left relative to the ascending trajectory along the vertical, and in the distance from the vertical. By means of the described apparatus it is possible to simulate the service in playing tennis from different heights and directions, depending on the row of emitters 15 that are activated in the arms of the cross. The heights can be for instance those typical of the strongest players in the world and the activated emitters 15 can be the left ones or the right ones with respect to the vertical ascending trajectory of the ball.

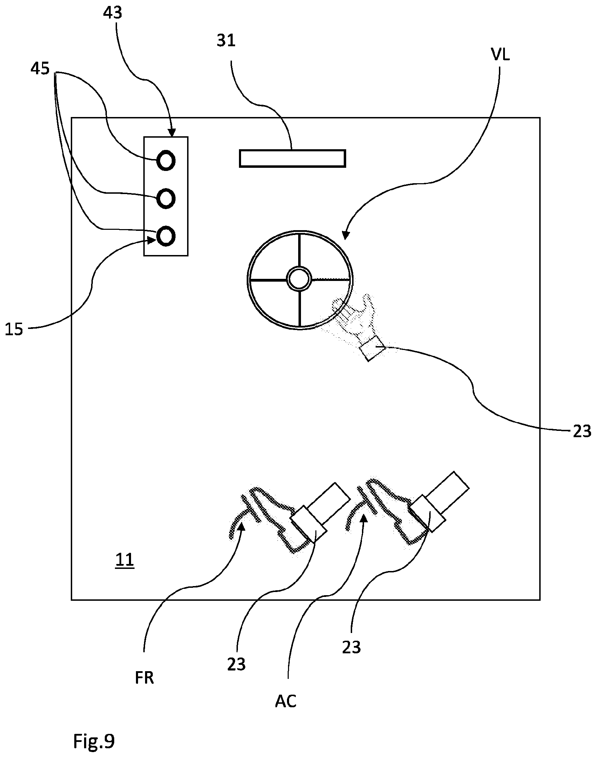

Referring to Figure 9, there is illustrated a third embodiment of the apparatus 11 according to the invention. The apparatus 11 of Figure 9 is intended especially for exercising individuals in driving vehicles. The vehicles can be of any kinds, terrestrial vehicles, naval vehicles or airborne vehicles. The apparatus of Figure 9 is intended especially for training an individual's response to events that may occur when driving a vehicle. In this embodiment, the apparatus comprises a plurality of light emitters 15. In the shown example, the emitters 15 are distributed in a linear form and represent corresponding colors 43 of a traffic light device 45. In the illustrated apparatus, the event is signaled by one of the emitters 15. In this embodiment the sequences Q stored in the memory unit 21 differ in the duration of the time interval elapsing between the activation of an emitter 15 and the activation of the subsequent emitter. For instance, in the case of a traffic light device 45, the sequences Q preferably determine the duration of the green, yellow and red lights and the time intervals elapsing between the switching on of a color and the switching on of the subsequent color. Similarly to the already described embodiments, the apparatus 11 may include a monocular or binocular "Eyetracker" sensor 31 for detecting eye movements, said sensor being capable of generating an electrical signal indicative of the eye movements of an individual 13. The sensor 31 is preferably of the binocular type, i.e. capable of detecting the eye movements of both eyes. The sensor 31 can be of the wireless type or of the wired type. The sensor 31 may further be associated to the traffic light device 45 or be separated from it. In other embodiments, the sensor can consist of glasses of the "Eyetracker" type worn by the individual using the apparatus 11. The apparatus 11 is further preferably equipped with at least one motion sensor 23. For instance, motion sensors 23 are preferably associated to the wrist of the arm of a user who normally holds a steering wheel "VL" and to either of the ankles, typically the right ankle usually acting on the brake pedal "FR" and the accelerator pedal "AC".

By means of the described apparatus it is possible to simulate different kinds of events occurring when driving a vehicle, by using emitters 15 with appropriate forms, for instance in the form of a dummy approaching the carriageway, for simulating crossing of a pedestrian, or of advancing vehicles, for simulating the presence of movable obstacles, or of any stationary objects such as buildings, plants, barriers etc., for simulating the presence of stationary obstacles.

### Industrial Applicability

The invention, though not being limited to this field, finds application particularly in the field of apparatuses for detecting, diagnosing and exercising the functionalities of the visual brain areas of individuals such as athletes practicing sports, especially tennis, at a competitive level.

The invention as described and illustrated may undergo several changes and modifications falling within the same inventive principle.

## Claims

1. Apparatus (11) for detecting, diagnosing and exercising an individual's functionalities, comprising:
- a plurality of emitters (15) emitting optical stimuli perceivable by an individual (13);
- a control unit (19) adapted to control the activation of said emitters (15) and programmed for activating said emitters (15) according to at least one parameter (P) and in accordance with a predetermined sequence (Q);
- a memory unit (21) in which predetermined sequences (Q) for the activation of said emitters (15) are stored, each sequence (Q) being identified by a univocal sequence reference and comprising the references of a group of emitters (15) belonging to said plurality of emitters and arranged along a path corresponding to the movement to be induced in the eyes of said individual (13);
- a sensor (31) "Eyetracker" capable of generating an electrical signal indicative of eye movements for detecting eye movements of an individual (13);
- a processing unit (25) operationally connected to said control unit (19) and programmed for processing said signal, for calculating the time (Tc) elapsed between the activation of an emitter (15) and the moment at which the individual's eyes become fixed on said emitter (15) of the sequence, **characterized in that**:
- said processing unit is further programmed for comparing the time (Tc) with a predetermined threshold (S₁) and for selecting, according to the value resulting from said comparison, the value of said at least one parameter (P) and a predetermined sequence (Q) out of said stored sequences (Q) to be performed in a subsequent working cycle of the apparatus;
- the sequences (Q) stored in the memory unit (21) are classified on the basis of a grade of difficulty in performance by an individual, and
- the processing unit (25) is programmed for selecting a sequence (Q) of lower difficulty than the performed sequence when Tc > S₁ and for selecting a sequence (Q) of higher difficulty than the performed sequence when Tc < S₁.

2. Apparatus according to claim 1, wherein the processing unit (25) is programmed for selecting the value of said at least one parameter (P) and the predetermined sequence (Q) so as to reach T_{c} = S₁.

3. Apparatus according to claim 1 or 2, wherein the apparatus (11) is equipped with a motion sensor (23) capable of generating an electrical signal indicative of the variation of the position of a body movable in space.

4. Method of controlling an apparatus (11) for detecting, diagnosing and exercising an individual's functionalities, comprising:
- a plurality of emitters (15) emitting optical stimuli perceivable by an individual (13);
- a control unit (19) adapted to control the activation of said emitters (15) and programmed for activating said emitters (15) according to at least one parameter (P) and in accordance with a predetermined sequence (Q);
- a memory unit (21) in which predetermined sequences (Q) for the activation of said emitters (15) are stored, each sequence (Q) being identified by a univocal sequence reference and comprising the references of a group of emitters (15) belonging to said plurality of emitters and arranged along a path corresponding to the movement to be induced in the eyes of said individual (13);
- a sensor (31) "Eyetracker" capable of generating a signal indicative of eye movements for detecting eye movements of an individual (13);
- a processing unit (25) operationally connected to said control unit (19), wherein a step is provided of:
- controlling the activation of said emitters (15) according to at least one parameter (P) and in accordance with a predetermined sequence (Q); **characterized in that** the following steps are provided:
- comparing with a predetermined first threshold (S₁) the time (Tc) elapsed between the activation of an emitter (15) and the moment at which the individual's eyes become fixed on said emitter (15) of the sequence (Q);
- selecting at least one parameter (P) and a predetermined sequence (Q) to be performed in a subsequent working cycle of the apparatus (11), out of said sequences stored in the memory unit (21), according to the result of said first comparison, and **in that** the sequences (Q) stored in the memory unit (21) are classified on the basis of a grade of difficulty in performance by an individual, and the processing unit (25) is programmed for selecting a sequence (Q) of lower difficulty than the performed sequence when Tc > S₁ and for selecting a sequence (Q) of higher difficulty than the performed sequence when Tc < S₁.

5. Method according to claim 4, wherein the apparatus further comprises a body motion sensor (23) capable of generating an electrical signal indicative of the variation of the position of a body movable in space and therefore of providing a signal indicative of the beginning and end of a body movement and wherein the following steps are provided:
- comparing with a predetermined second threshold (S₂) the time (Ts) elapsed between the activation of an emitter (15) and the moment at which the individual's movement begins or a specific movement of the individual prescribed by the exercise being performed is detected;
- selecting at least one parameter (P) and a predetermined sequence (Q) to be performed in a subsequent working cycle of the apparatus, out of said sequences (Q) stored in the memory unit (21), according to the result of said second comparison.

6. Method according to claim 4 or 5, wherein there is provided the step of varying, as a result of said first or, when depending from claim 5, second comparison, said at least one parameter (P) while maintaining the same sequence (Q) as in the preceding cycle, or of varying, as a result of the comparison, the sequence (Q) while maintaining said at least one parameter (P) of the preceding cycle.

7. Method according to claim 4 or 5, wherein there is provided the step of varying, as a result of said first or, when depending from claim 5, second comparison, said at least one parameter (P) and the sequence (Q) with respect to the preceding cycle.

8. Method according to claim 6, wherein there is provided the step of varying the value of said at least one parameter (P) so as to reduce the performing speed of the sequence when Tc > S₁ and so as to increase the performing speed of the sequence when Tc < S₁.

9. Method according to claim 8, wherein the value of said at least one parameter (P) is varied by a value such that T_{C} = S₁.

## Patentansprüche

1. Apparat (11) zur Erfassung, Diagnose und Ausübung der Funktionen eines Individuums, umfassend:
- eine Mehrzahl von Emittern (15) optischer Reize, die von einem Individuum (13) wahrnehmbar sind;
- eine Steuereinheit (19) zur Aktivierung der Emitter (15), die zur Aktivierung der Emitter (15) entsprechend wenigstens einem Parameter (P) gemäß einer vorgegebenen Sequenz (Q) programmiert ist;
- eine Speichereinheit (21), in der vorgegebene Sequenzen (Q) zur Aktivierung der Emitter (15) gespeichert sind, wobei jede Sequenz (Q) durch einen eindeutigen Sequenzbezug gekennzeichnet ist und die Erkennungsbezüge einer Gruppe von Emittern (15) umfasst, die zu der Mehrzahl von Emittern gehören und entlang einer der Bewegung entsprechenden Trajektorie angeordnet sind, die in den Augen des Individuums (13) induziert werden soll;
- einen "Eyetracker"-Sensor (31), der in der Lage ist, ein elektrisches Signal zu erzeugen, das Augenbewegungen anzeigt, um Augenbewegungen eines Individuums (13) zu erfassen;
- eine Verarbeitungseinheit (25), die mit der Steuereinheit (19) wirkverbunden und zur Signalverarbeitung programmiert ist, um den Zeitraum (Tc) zwischen der Aktivierung eines Emitters (15) und dem Moment zu berechnen, in dem die Augen des Individuums fest auf den Emitter (15) der Sequenz gerichtet sind,
**dadurch gekennzeichnet, dass**
die Verarbeitungseinheit ferner programmiert ist, um die Zeit (Tc) mit einem vorgegebenen Schwellenwert (S1) zu vergleichen und auf der Grundlage des aus dem Vergleich hervorgehenden Wertes den Wert des wenigstens eines Parameters (P) und eine vorgegebene Sequenz (Q) aus den gespeicherten Sequenzen (Q) auszuwählen, die in einem nachfolgenden Arbeitszyklus des Apparats auszuführen sind;
die in der Speichereinheit (21) gespeicherten Sequenzen (Q) nach dem Schwierigkeitsgrad der Ausführung seitens eines Individuums eingestuft sind und
die Verarbeitungseinheit (25) programmiert ist, um eine Sequenz (Q) geringerer Schwierigkeit als die ausgeführten Sequenz auszuwählen, wenn Tc > S1 ist bzw. eine Sequenz (Q) größerer Schwierigkeit als die ausgeführte Sequenz auszuwählen, wenn Tc < S1 ist.

2. Apparat nach Anspruch 1, wobei die Verarbeitungseinheit (25) programmiert ist, um den Wert des wenigstens einen Parameters (P) und die vorgegebene Sequenz (Q) so auszuwählen, dass Tc = S1 ist.

3. Apparat nach Anspruch 1 oder 2, wobei der Apparat (11) mit einem Bewegungssensor (23) ausgestattet ist, der in der Lage ist, ein elektrisches Signal zu erzeugen, das die Änderung der Position eines im Raum beweglichen Körpers anzeigt.

4. Verfahren zur Steuerung eines Apparats (11) zur Erfassung, Diagnose und Ausübung der Funktionen eines Individuums, umfassend:
- eine Mehrzahl von Emittern (15) optischer Reize, die von einem Individuum (13) wahrnehmbar sind;
- eine Steuereinheit (19) zur Aktivierung der Emitter (15), die zur Aktivierung der Emitter (15) entsprechend wenigstens einem Parameter (P) gemäß einer vorgegebenen Sequenz (Q) programmiert ist;
- eine Speichereinheit (21), in der vorgegebene Sequenzen (Q) zur Aktivierung der Emitter (15) gespeichert sind, wobei jede Sequenz (Q) durch einen eindeutigen Sequenzbezug gekennzeichnet ist und die Erkennungsbezüge einer Gruppe von Emittern (15) umfasst, die zu der Mehrzahl von Emittern gehören und entlang einer der Bewegung entsprechenden Trajektorie angeordnet sind, die in den Augen des Individuums (13) induziert werden soll;
- einen "Eyetracker"-Sensor (31), der in der Lage ist, ein Signal zu erzeugen, das Augenbewegungen anzeigt, um Augenbewegungen eines Individuums (13) zu erfassen;
- eine Verarbeitungseinheit (25), die mit der Steuereinheit (19) wirkverbunden ist, wobei folgende Schritte vorgesehen sind:
- Steuern der Aktivierung der Emitter (15) entsprechend wenigstens einem Parameter (P) gemäß einer vorgegebenen Sequenz (Q);
- Vergleichen des Zeitraums (Tc) zwischen der Aktivierung eines Emitters (15) und dem Moment, in dem die Augen des Individuums fest auf den Emitter (15) der Sequenz (Q) gerichtet sind, mit einem vorgegebenen Schwellenwert (S1);
- Auswählen wenigstens eines Parameters (P) und einer vorgegebenen, in einem nachfolgenden Arbeitszyklus des Apparats (11) auszuführenden Sequenz (Q) aus den in der Speichereinheit (21) gespeicherten Sequenzen auf der Grundlage des Ergebnisses des ersten Vergleichs und die in der Speichereinheit (21) gespeicherten Sequenzen (Q) sind nach dem Schwierigkeitsgrad der Ausführung seitens eines Individuums eingestuft und die Verarbeitungseinheit (25) ist programmiert, um eine Sequenz (Q) geringerer Schwierigkeit als die ausgeführte Sequenz auszuwählen, wenn Tc > S1 ist bzw. um eine Sequenz (Q) größerer Schwierigkeit als die ausgeführte Sequenz auszuwählen, wenn Tc < S1 ist.

5. Verfahren nach Anspruch 4, wobei der Apparat ferner einen Körperbewegungssensor (23) umfasst, der in der Lage ist, ein elektrisches Signal zu erzeugen, das die Änderung der Position eines im Raum beweglichen Körpers anzeigt und ein Signal bereitzustellen, das den Anfang und das Ende einer Körperbewegung anzeigt, wobei die folgenden Schritte vorgesehen sind:
- Vergleichen des Zeitraums (Ts) zwischen der Aktivierung eines Emitters (15) und dem Moment, in dem die Bewegung des Individuums beginnt oder eine in der auszuführenden Übung festgelegte, spezifische Bewegung des Individuums erfasst wird, mit einem zweiten vorgegebenen Schwellenwert (S2);
- Auswählen wenigstens eines Parameters (P) und einer vorgegebenen, in einem nachfolgenden Arbeitszyklus des Apparats auszuführenden Sequenz (Q) aus den in der Speichereinheit (21) gespeicherten Sequenzen (Q) auf der Grundlage des Ergebnisses des zweiten Vergleichs.

6. Verfahren nach Anspruch 4 oder 5, wobei der Schritt vorgesehen ist, auf der Grundlage des ersten Vergleichs oder - wenn abhängig von Anspruch 5 - des zweiten Vergleichs den wenigstens einen Parameter (P) unter Beibehaltung der Sequenz (Q) des vorhergehenden Zyklus zu ändern oder auf der Grundlage des Vergleichs unter Beibehaltung des wenigstens einen Parameters (P) des vorhergehenden Zyklus die Sequenz (Q) zu ändern.

7. Verfahren nach Anspruch 4 oder 5, wobei der Schritt vorgesehen ist, auf der Grundlage des Vergleich des ersten oder - wenn abhängig von Anspruch 5 - des zweiten Vergleichs den wenigstens einen Parameter (P) und die Sequenz (Q) gegenüber dem vorhergehenden Zyklus zu ändern.

8. Verfahren nach Anspruch 6, wobei der Schritt vorgesehen ist, den Wert des wenigstens einen Parameters (P) zu ändern, um die Ausführungsgeschwindigkeit der Sequenz zu vermindern, wenn Tc > S1 ist, bzw. die Ausführungsgeschwindigkeit der Sequenz zu erhöhen, wenn Tc < S1 ist.

9. Verfahren nach Anspruch 8, wobei der Wert des wenigstens einen Parameters (P) um einen derartigen Wert geändert wird, dass Tc = S1 ist.

## Revendications

1. Appareil (11) pour la détection, le diagnostic et l'exercice des fonctions d'un individu comprenant :
- une pluralité d'émetteurs (15) de stimulations optiques perceptibles par un individu (13) ;
- une unité (19) de commande de l'activation des dits émetteurs (15), programmée pour activer lesdits émetteurs (15) en fonction d'au moins un paramètre (P) et en accord avec une séquence prédéfinie (Q) ;
- une unité (21) de mémoire où sont sauvegardées des séquences (Q) prédéfinies d'activation des dits émetteurs (15), chaque séquence (Q) étant identifiée par une référence univoque de séquence et comprenant des références d'identification d'un groupe d'émetteurs (15) appartenant à ladite pluralité d'émetteurs disposés le long d'une trajectoire correspondante au mouvement que l'on souhaite induire dans les yeux du dit individu (13) ;
- un capteur (31) « Eyetracker » susceptible de générer un signal électrique indicatif des mouvements oculaires pour la détection des mouvements oculaires d'un individu (13) ;
- une unité (25) de traitement fonctionnellement associée à ladite unité (19) de commande et programmée pour traiter ledit signal, pour calculer le temps (T_{c}) qui s'est écoulé entre l'activation d'un émetteur (15) et l'instant où les yeux de l'individu se sont fixés sur ledit émetteur (15) de la séquence,
**caractérisé en ce que**
ladite unité de traitement est également programmée pour comparer le temps (T_{c}) avec un seuil (S₁) prédéfini et pour sélectionner, en fonction de la valeur qui résulte de ladite comparaison, la valeur d'au moins un paramètre (P) et une séquence (Q) prédéfinie parmi lesdites séquences (Q) sauvegardées à effectuer pendant un cycle successif de fonctionnement de l'appareil ;
les séquences (Q) sauvegardées dans l'unité de mémoire (21) sont classées sur la base d'un niveau de difficulté d'exécution d'un individu, et
l'unité de traitement (25) est programmée pour sélectionner une séquence (Q) de difficulté plus basse par rapport aux séquences effectuées lorsque T_{c} > S₁ et pour sélectionner une séquence (Q) de difficulté plus élevée par rapport aux séquences effectuées lorsque T_{c} < S₁.

2. Appareil selon la revendication 1, où l'unité (25) de traitement est programmée pour sélectionner la valeur de dit au moins un paramètre (P) et la séquence (Q) prédéfinie, de manière à atteindre T_{c} = S₁.

3. Appareil selon la revendication 1 ou 2, où l'appareil (11) est équipé d'un capteur (23) de mouvement, susceptible de générer un signal électrique indicatif de la variation de position d'un corps qui peut se déplacer dans l'espace.

4. Méthode de contrôle d'un appareil (11) pour la détection, le diagnostic et l'exercice des fonctions d'un individu comprenant :
- une pluralité d'émetteurs (15) de stimulations optiques perceptibles par un individu (13) ;
- une unité (19) de commande apte à l'activation des dits émetteurs (15), programmée pour activer lesdits émetteurs (15) en fonction d'au moins un paramètre (P) et en accord avec une séquence prédéfinie (Q) ;
- - une unité (21) de mémoire où sont sauvegardées des séquences (Q) prédéfinies d'activation des dits émetteurs (15), chaque séquence (Q) étant identifiée par une référence univoque de séquence et comprenant des références d'identification d'un groupe d'émetteurs (15) appartenant à ladite pluralité d'émetteurs disposés le long d'une trajectoire correspondante au mouvement que l'on souhaite induire dans les yeux du dit individu (13) ;
- un capteur (31) « Eyetracker » susceptible de générer un signal indicatif des mouvements oculaires pour la détection des mouvements oculaires d'un individu (13) ;
- une unité (25) de traitement fonctionnellement associée à ladite unité (19) de commande, où une phase est prévue pour :
- commander l'activation des dits émetteurs (15) en fonction d'au moins un paramètre (P) et en accord avec une séquence prédéfinie ; **caractérisée en ce que** lesdites phases sont prévues pour :
- comparer avec un seuil (S₁) prédéfini le temps (T_{c}) qui s'est écoulé entre l'activation d'un émetteur (15) et l'instant où les yeux de l'individu se sont fixés sur ledit émetteur (15) de la séquence (Q) ;
- sélectionner au moins un paramètre (P) et une séquence (Q) prédéfinie à effectuer pendant un cycle successif de fonctionnement de l'appareil (11), parmi lesdites séquences sauvegardées dans l'unité (21) de mémoire, en fonction du résultat de ladite première comparaison, et les séquences (Q) sauvegardées dans l'unité de mémoire (21) sont classées sur la base d'un niveau de difficulté d'exécution d'un individu, et l'unité de traitement (25) est programmée pour sélectionner une séquence (Q) de difficulté plus basse par rapport aux séquences effectuées lorsque T_{c} > S₁ et pour sélectionner une séquence (Q) de difficulté plus élevée par rapport aux séquences effectuées lorsque T_{c} < S₁.

5. Méthode selon la revendication 4, où l'appareil comprend également un capteur (23) de mouvement du corps, susceptible de générer un signal électrique indicatif de la variation de position d'un corps qui se déplace dans l'espace et fournir un signal indicatif du début et de la fin du mouvement du corps et où les phases suivantes sont prévues :
- comparer avec un deuxième seuil (S₂) prédéfini le temps (Tₛ) qui s'est écoulé entre l'activation d'un émetteur (15) et l'instant où commence le mouvement de l'individu ou qu'un mouvement spécifique de l'individu, établi par l'exercice en exécution, est détecté ;
- sélectionner au moins un paramètre (P) et une séquence (Q) prédéfinie à exécuter lors d'un cycle successif de fonctionnement de l'appareil, entre lesdites séquences (Q) sauvegardées dans l'unité (21) de mémoire, en fonction du résultat de ladite seconde comparaison.

6. Méthode selon la revendication 4 ou 5, où est prévue la phase de varier, en tant que résultat de la première ou, quand dépendante de la revendication 5, la seconde comparaison, ledit au moins un paramètre (P) en maintenant la même séquence (Q) du cycle précédent, ou de varier, suite à la comparaison, la séquence (Q) en maintenant ledit au moins un paramètre (P) du cycle précédent.

7. Méthode selon la revendication 4 ou 5, où est prévue la phase de varier, comme résultat de ladite première ou, quand dépendante de la revendication 5, la seconde comparaison, ledit au moins un paramètre (P) et la séquence (Q) par rapport au cycle précédent.

8. Méthode selon la revendication 6, où est prévue la phase de varier la valeur du dit au moins un paramètre (P) de manière à réduire la vitesse d'exécution de la séquence lorsque T_{c} > S₁ et de manière à augmenter la vitesse d'exécution de la séquence lorsque T_{c} < S₁.

9. Méthode selon la revendication 8, où la valeur du dit au moins un paramètre (P) est varié d'une valeur telle que T_{c} = S₁.
